# EUROPEAN PATENT APPLICATION

(11) **EP 4 809 919 A2**
(43) Date of publication of application: **23.09.2026**
(21) Application number: 26165327.3
(22) Date of filing: 17.03.2026
(51) Int. Cl.: A61B 5/145, A61B 5/00

(54) **APPARATUS FOR PROVIDING INFORMATION AND METHOD THEREOF**

(30) Priority: 20.03.2025 KR 20250036136
(71) Applicant: I-SENS, INC., Seoul 06646 (KR)
(72) Inventor: MOK, Jin Won, 06646 Seoul (KR); SEO, Jung Hee, 06646 Seoul (KR); KIM, Hae Na, 06646 Seoul (KR)
(74) Representative: BCKIP Part mbB

(57) **Abstract**

Provided is a method for providing information performed by an electronic apparatus, the method including providing first biometric data of a user of a sensor, determining, subsequent to providing the first biometric data, a noise segment in the first biometric data based on a sensor sensitivity, and providing, based on the determination of the noise segment, second biometric data in which at least one value corresponding to the noise segment in the first biometric data is modified.

## Description

### CROSS-REFERENCE TO RELATED APPLICATION(S)

This application claims the benefit of Korean Patent Application No. 10-2025-0036136, filed on March 20, 2025, in the Korean Intellectual Property Office.

### FIELD OF THE INVENTION

Example embodiments relate to an apparatus for providing information and a method thereof. More specifically, the example embodiments relate to an electronic device for providing biometric data and generating a modified version thereof by modifying biometric data corresponding to a noise segment, and a method thereof.

### DESCRIPTION OF THE RELATED ART

There is a significant clinical need for monitoring various physiological parameters, such as blood glucose, to prevent, diagnose, and treat diseases. For example, diabetic patients often require insulin administration to maintain glycemic control, making continuous glucose monitoring essential. Furthermore, for individuals not yet diagnosed with diabetes, regular monitoring of blood glucose levels serves as a critical tool for health management and early disease prevention.

Conventionally, invasive blood glucose meters (e.g., finger-stick meters) have been the primary tool for glycemic measurement. However, such invasive meters have inherent limitations, including an inability to provide continuous monitoring as they only capture point-in-time measurements at the moment of sampling. Furthermore, the requirement for frequent blood sampling throughout the day, necessitated by the dynamic fluctuations in blood glucose levels, imposes a significant physical and psychological burden on the user.

To address the limitations of invasive monitoring, continuous glucose monitoring systems (CGMS) have been developed, requiring sophisticated calibration techniques to ensure high clinical accuracy in detecting blood glucose through the CGMS.

In particular, when a noise segment in which a sensor of the CGMS is determined to be in a compressed state is determined, it is necessary to modify and regenerate the biometric data.

In this regard, related documents such as KR102623744B1or KR101278605B1 may be referred to.

### SUMMARY OF THE INVENTION

An aspect provides an electronic device configured to modify biometric data corresponding to a noise segment determined to be in a compressed stated prior to providing the information, and a method thereof.

The goals to be achieved by example embodiments of the present disclosure are not limited to the object described above, and other objects may be inferred from the following example embodiments.

According to an aspect, there is provided a method for providing information performed by an electronic apparatus, the method including providing first biometric data of a user of a sensor, determining , subsequent to providing the first biometric data, a noise segment in the first biometric data based on a sensor sensitivity, and providing, based on the determination of the noise segment, second biometric data in which at least one value corresponding to the noise segment in the first biometric data is modified.

The noise segment may correspond to a segment in which the sensor is determined to be in a compressed state.

The noise segment determined to be in the compressed state may correspond to a segment in which the sensor sensitivity is maintained at or below a reference value for a reference duration or longer.

The reference value and the reference duration may be adaptively adjusted in view of a disease severity level determined based on historical biometric data of the user.

The providing of the second biometric data may include modifying the at least one value corresponding to the noise segment in the first biometric data via interpolation, and providing second biometric data including the at least one value modified via the interpolation in the noise segment.

The providing of the second biometric data may include providing second biometric data in which a representation method for distinguishing the noise segment from a segment outside the noise segment in the first biometric data is applied to the noise segment.

The providing of the second biometric data may include providing a notification to the user when a disease severity level determined based on historical biometric data of the user is at or above a reference level.

The providing of the notification may include providing a notification to the user when the disease severity level determined based on the historical biometric data of the user is at or above the reference level and at least one modified value in the second biometric data is at or above a threshold value.

The sensor sensitivity may be determined based on a function defining a relationship between an electrical current value measured from blood of the user and a blood glucose level corresponding to the electrical current value.

The determining of the noise segment may include determining the noise segment in the first biometric data in accordance with a predetermined cycle set in view of a disease severity level determined based on historical biometric data of the user.

According to another aspect, there is provided an electronic device for providing information, the apparatus including a processor and at least one memory configured to store at least one instruction. When the at least one instruction is executed, the processor may be configured to provide first biometric data of a user of a sensor, determine, subsequent to providing the first biometric data, a noise segment in the first biometric data based on a sensor sensitivity, and provide, based on the determination of the noise segment, second biometric data in which at least one value corresponding to the noise segment in the first biometric data is modified.

For example, the memory may store instructions that are configured to, when executed by the processor, to cause the processor to carry out one or more of the steps described above.

The noise segment may correspond to a segment in which the sensor is determined to be in a compressed state.

The noise segment determined to be in the compressed state may correspond to a segment in which the sensor sensitivity is maintained at or below a reference value for a reference duration or longer.

The reference value and the reference duration may be adaptively adjusted in view of a disease severity level determined based on historical biometric data of the user.

The processor, when providing the second biometric data, may be configured to modify the at least one value corresponding to the noise segment in the first biometric data via interpolation and provide second biometric data including the at least one value modified via the interpolation in the noise segment.

The processor, when providing the second biometric data, may be configured to provide second biometric data in which a representation method for distinguishing the noise segment from a segment outside the noise segment in the first biometric data is applied to the noise segment.

The sensor sensitivity may be determined based on a function defining a relationship between an electrical current value measured from blood of the user and a blood glucose level corresponding to the electrical current value.

The processor, when determining the noise segment, may be configured to determine the noise segment in the first biometric data in accordance with a predetermined cycle set in view of a disease severity level determined based on historical biometric data of the user.

According to still another aspect, there is also provided a method for providing information performed by a server, the method including identifying a condition applied for determination of a noise segment, the condition requiring a sensor sensitivity is maintained at or below a reference value for a reference duration or longer, adaptively adjusting the condition in view of a disease severity level of a user, and providing the adaptively adjusted condition to a corresponding user terminal.

Detailed descriptions of other example embodiments are included in the detailed description and drawings.

Additional aspects of example embodiments will be set forth in part in the description which follows and, in part, will be apparent from the description, or may be learned by practice of the disclosure.

According to example embodiments, it is possible to provide biometric data by modifying biometric data corresponding to a noise segment determined to be in a compressed state.

Effects of the present disclosure are not limited to those described above, and other effects may be made apparent to those skilled in the art from the following description.

### BRIEF DESCRIPTION OF THE DRAWINGS

These and/or other aspects, features, and advantages of the invention will become apparent and more readily appreciated from the following description of example embodiments, taken in conjunction with the accompanying drawings of which:
FIG. 1 is a schematic block diagram illustrating a system for acquiring a blood glucose level, blood glucose data, or sensor data according to an example embodiment;
FIG. 2 is a diagram illustrating a user device according to an example embodiment;
FIG. 3 is a flowchart illustrating a method for providing information according to an example embodiment;
FIG. 4 is a flowchart illustrating a method for providing information performed by a server according to an example embodiment;
FIG. 5 is a flowchart illustrating a method for providing information performed by a user device according to an example embodiment;
FIG. 6 is a flowchart illustrating a method for providing information performed by a user device according to another example embodiment;
FIG. 7 is a flowchart illustrating a process of providing a notification by a user device according to an example embodiment;
FIG. 8 is a flowchart illustrating a method for providing information performed by a server according to an example embodiment; and
FIG. 9 is a diagram illustrating a relationship between a server and a user device according to an example embodiment.

### DETAILED DESCRIPTION OF EXEMPLARY EMBODIMENTS OF THE INVENTION

Terms used in the example embodiments are selected, as much as possible, from general terms that are widely used at present while taking into consideration the functions obtained in accordance with the present disclosure, but these terms may be replaced by other terms based on intentions of those skilled in the art, customs, emergence of new technologies, or the like. Also, in a particular case, terms that are arbitrarily selected by the applicant of the present disclosure may be used. In this case, the meanings of these terms may be described in corresponding description parts of the present disclosure. Accordingly, it should be noted that the terms used herein should be construed based on practical meanings thereof and the whole content of this specification rather than being simply construed based on names of the terms.

In the entire specification, when an element is referred to as "comprising" or "including" another element, the element should not be understood as excluding other elements as long as there is no special conflicting description, and the element may include at least one other element. In addition, the terms "unit" and "module", for example, may refer to a component that exerts at least one function or operation, and may be realized in hardware or software, or may be realized by combination of hardware and software.

Throughout the specification, expression "at least one of a, b, and c" may include 'a only', 'b only', 'c only', 'a and b', 'a and c', 'b and c', or 'all of a, b, and c'.

The "terminal" referred hereinafter may be embodied as a computer of a portable device that can access a server or another terminal through a network. In the present disclosure, a computer may include, for example, a notebook computer, a desktop computer, and a laptop equipped with a web browser, and a portable device, for example, as a wireless communication device that guarantees portability and mobility, may include all kinds of handheld wireless communication devices such as a communication-based terminal, a smartphone, and tablet PC, supporting international mobile telecommunication (IMT), code division multiple access (CDMA), w-code division multiple access (W-CDMA), long-term evolution (LTE), and the like.

In the following description, example embodiments of the present disclosure will be described in detail with reference to the drawings so that those skilled in the art can easily carry out the present disclosure. The present disclosure may be applied in many different forms and is not limited to the embodiments described herein.

Hereinafter, example embodiments of the present disclosure will be described in detail with reference to the accompanying drawings.

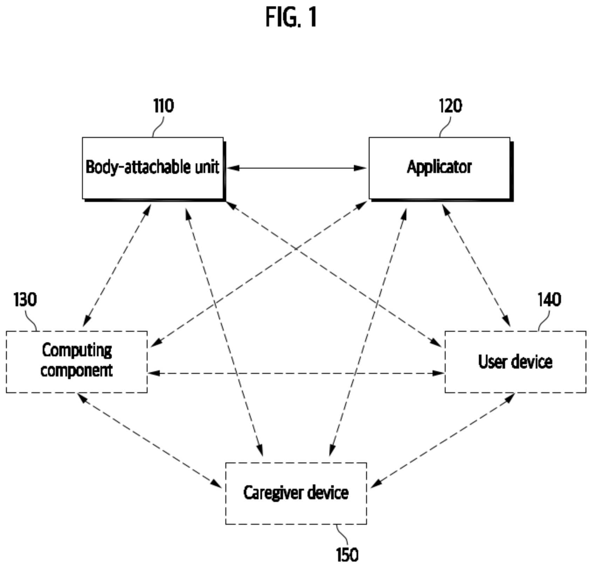

FIG. 1 is a schematic block diagram illustrating a system for acquiring a blood glucose level, blood glucose data, or sensor data according to an example embodiment.

According to various example embodiment, a blood glucose acquisition system may include a body-attachable unit 110 and an applicator 120. According to an example embodiment, the blood glucose acquisition system may further include one or more of a computing component 130, a user device 140, and a caregiver device 150. According to an example embodiment, the blood glucose acquisition system may further include a network infrastructure supporting information transmission and reception between at least two or more of the body-attachable unit 110, the applicator 120, the computing component 130, the user device 140, the caregiver device 150, and an external device.

The body-attachable unit 110, the applicator 120, the user device 140, and the caregiver device 150 may include a memory and a processor, and may also include a transceiver. Additionally, each of the body-attachable unit 110, the applicator 120, the computing component 130, the user device 140, and the caregiver device 150 may refer to a unit that processes at least one function or operation, and may be implemented in hardware, software, or a combination of hardware and software.

Although the body-attachable unit 110, the applicator 120, the computing component 130, the user device 140, and the caregiver device 150 are referred to as separate devices, servers, or components in the example embodiments of the present disclosure, they may represent logically divided structures, and at least one of them may be implemented by a function separated from a single device or a single server. For example, in the present disclosure, the computing component 130 and the user device 140 may be integrated into a single device. In this case, some of the processes described as transmission and reception of information between the computing component 130 and the user device 140 may actually be a data exchange process performed within the single device. Additionally, the user device 140 and the caregiver device 150 may be integrated into a single device, and information including the user's sensor data and the like may be transmitted and received via a server or cloud, but this is merely an example. As another example, the computing component 130, which may include at least one of hardware and software, may be incorporated into the body-attachable unit 110 (e.g., by downloading software corresponding to the computing component 130 onto the body-attachable unit 110 via a web or an application store), or coupled to the body-attachable unit 110 (e.g., through mutual connection via a USB port). As yet another example, the computing component 130 may be implemented as hardware or software of an external device.

According to an example embodiment, the body-attachable unit 110, the applicator 120, the computing component 130, the user device 140, and the caregiver device 150 may include multiple computer systems or computer software implemented as network servers. For example, at least one of the body-attachable unit 110, the applicator 120, the computing component 130, the user device 140, and the caregiver device 150 may refer to a computer system and computer software connected to a sub-device capable of communicating with a computer network such as intranet and internet, and configured to receive a task execution request and output a task execution result. Additionally, at least one of the body-attachable unit 110, the applicator 120, the computing component 130, the user device 140, and the caregiver device 150 may be understood in a broad sense as a series of application programs operating on a network server and various databases established within or connected to other nodes. For example, at least one of the body-attachable unit 110, the applicator 120, the computing component 130, the user device 140, and the caregiver device 150 may be implemented using various network server programs that are provided according to operating systems such as DOS, Windows, Linux, UNIX, or MacOS.

Meanwhile, although each operational entity is referred to as the body-attachable unit 110, the applicator 120, the user device 140, or the caregiver device 150 for convenience of description, they should be understood as comprehensive devices corresponding to, including, or included in various types of devices such as computer devices and mobile communication terminals.

The body-attachable unit 110 may be a device configured to process and provide various types of information and includes a sensor having at least a portion configured to be inserted into the body of a user. The sensor may be provided to the user in a pre-assembled state within the body-attachable unit 110. Alternatively, the sensor may be provided separately from the body-attachable unit 110 and configured to be coupled to the body-attachable unit 110 by the user or via an operation of the applicator 120. The body-attachable unit 110 may be attached to the body of the user and perform various operations such as acquiring blood glucose or sensor data from the user's bodily fluid via the sensor. The bodily fluid utilized by the body-attachable unit 110 to perform operations such as acquiring blood glucose may include interstitial fluid. However, this is merely an example, and the method described in the present disclosure is not limited thereto. According to the method of the present disclosure, since the body-attachable unit 110 remains attached to the body of the user, blood glucose acquisition may be performed at shorter intervals compared to conventional methods requiring blood sample collection.

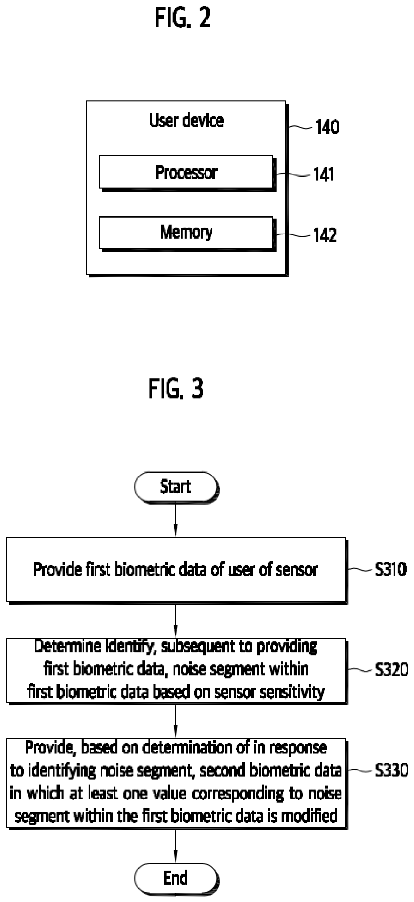

According to an example embodiment, the body-attachable unit 110 may acquire one or more pieces of information and data related to blood glucose of the user based on the one or more pieces of information (Although specific operational entities will be described later with reference to FIG. 2, various example embodiments regarding distribution of processing tasks may fall within the scope of the present disclosure. For example, an operation of calculating a blood glucose level may be performed by the computing component 130, and alternatively, the body-attachable unit 110 may perform at least one of operations while the computing component 130 performs the remaining operations). The one or more pieces of information acquired by the body-attachable unit 110 may include an electrical current value measured in response to the sensor contacting the interstitial fluid of the user. According to a more specific example embodiment, an enzyme provided in the sensor of the body-attachable unit 110 reacts with glucose present in the interstitial fluid of the user (e.g., glucose diffusing into the interstitial fluid), releasing electrons, and the body-attachable unit 110 may then acquire an electrical current value proportional the released electrons. Specific operations for acquiring the electrical current value proportional to the released electrons may include acquiring a digital signal based on the released electrons and sampling the digital signal. However, the scope of the present disclosure is not limited thereto.

The applicator 120 may be configured to facilitate the attachment of the body-attachable unit 110 to the body of the user. For example, the applicator 120 may be configured to eject the body-attachable unit 110 in an outward or downward direction when the applicator 120 is activated while the body-attachable unit 110 is seated within the applicator 120, and by directing the ejection toward the skin of the user, the body-attachable unit 110 may be attached to the body of the user. According to an example embodiment, the applicator 120 may include one or more of a plunger, a needle, a needle retraction mechanism, and a spring, and at least one of the needle and the needle retraction mechanism may facilitate smoother insertion of the sensor of the body-attachable unit 110 into the skin of the user. However, the specific operation and usage of the applicator 120 described herein are provided merely as an example of the present disclosure and shall not be construed to limit the scope of the present disclosure.

The computing component 130 may be a component operated and managed by various entities. The various entities may include one or more of the aforementioned "user" and a person involved in program development, but this is merely an example. For example, a user of the computing component 130 may include a technician, a researcher, medical personnel, or a guardian monitoring the blood glucose acquisition process.

The computing component 130 may be implemented as software. For example, the computing component 130 may correspond to a program (e.g., an application) whose function is performed by the body-attachable unit 110 or a program whose function is performed by the user device 140. However, the computing component 130 of the present disclosure may not necessarily be implemented as software, and various other example embodiments may fall in the scope of the present disclosure. For example, the computing component 130 may be implemented as a combination of hardware and software (more specifically, a device configured to execute software functions).

The computing component 130 may interact with an entity, such as the user, or with a device, such as the body-attachable unit 110, and may also perform operations related thereto. For example, the computing component 130 may receive an electrical current value from the body-attachable unit 110 and acquire a blood glucose level based on the received electrical current value. Additionally, the computing component 130 may determine or extract a conversion function utilized for acquiring the blood glucose level.

The user device 140 may be a device operated and managed by various entities. The various entities may include the aforementioned "user", but this is merely an example.

The user device 140 may interact with an entity such as the user and a device such as the body-attachable unit 110, and may also perform operations related thereto. For example, the user device 140 may be configured to provide a blood glucose level or blood glucose data to the user (e.g., by screen display). Also, the user device 140 may be configured to provide an alarm, a warning, or an instruction related to the blood glucose level to the user and receive feedback or commands from the user (e.g., through touch input) related to the blood glucose level. Additionally, the user device 140 may transmit feedback or commands to the body-attachable unit 110.

According to an example embodiment, the computing component 130 may be implemented as software, and the functionality of such software may be executed on the user device 140. Furthermore, to enable the software functionality to be executed on the user device 140, the computing component 130 may be incorporated into the user device 140 (e.g., by downloading software corresponding to the computing component 130 onto the user device 140 via a web or an application store) or coupled to the user device 140 (e.g., through mutual connection via a USB port).

The caregiver device 150 may be a device operated and managed by various entities. The various entities may include one or more of a medical personnel monitoring the condition of the user and a guardian of the user of the body-attachable unit 110, but this is merely an example. For example, depending on an example embodiment, an entity operating and managing the caregiver device 150 may include a person who monitors the blood glucose acquisition process, such as a technician or a researcher.

The caregiver device 150 may interact with an entity such as a manager or with a device such as the user device 140, and may also perform operations related thereto. For example, the caregiver device 150 may acquire health management information based on input from the caregiver and provide the acquired health management information to the user device 140. Also, the caregiver device 150 may provide information acquired from the computing component 130 or the user device 140 to the caregiver (e.g., by displaying it on a screen).

Details regarding operations of the body-attachable unit 110 and the computing component 130 will be provided below with reference to FIG. 2.

The body-attachable unit 110, the applicator 120, the user device 140, and the caregiver device 150 may include a computing device, a mobile communication terminal, a server, or the like. The body-attachable unit 110, the applicator 120, the user device 140, and the caregiver device 150 may include or be connected to an input device, such as a touch pad, a mouse, and a keyboard, to receive input. Additionally, the body-attachable unit 110, the applicator 120, the user device 140, and the caregiver device 150 may include or be connected to an output device, such as a display screen, a speaker, and an interface device, to provide information to a user. Furthermore, input and output devices of the body-attachable unit 110, the applicator 120, the user device 140, and the caregiver device 150 may be incorporated into or interrelated to one another. For example, an interface for receiving input may be displayed on at least one of the body-attachable unit 110, the applicator 120, the user device 140, and the caregiver device 150.

Operations related to a series of methods of providing information according to various example embodiments may be implemented by a single physical device or by multiple physical devices that are operatively coupled to one another. For example, at least one component included in the blood glucose acquisition system may be incorporated into one physical device, while the remaining components are incorporated into another physical device. For example, the one physical device may be incorporated as a part of the body-attachable unit 110, while another physical device may be incorporated as a part of the applicator 120 or another external device. In some cases, components included in the blood glucose acquisition system may be distributed across different physical devices, and the distributed components may be operatively coupled to perform a function and an operation of the blood glucose acquisition system. For example, the body-attachable unit 110 described in this specification may include at least one sub-device, and operations described as being performed by the body-attachable unit 110 may be performed by a first sub-device, while other operations may be performed by a second sub-device.

FIG. 2 is a diagram illustrating a user device according to an example embodiment. The user device 140 may correspond to an electronic device performing a method for providing information of the present disclosure.

Referring to FIG. 2, the user device 140 may include a processor 141 and a memory 142. The user device 140 shown in FIG. 2 illustrates only components related to the present embodiment. Therefore, it is to be understood by those skilled in the art that other general-purpose components may be further included in addition to the components illustrated in FIG. 2. For example, the electronic device 140 may include a communication device including at least one transceiver, an input unit, and an output unit. The communication device may communicate with an external electronic device as a device for wired/wireless communication. The external device may be a terminal or a server. In addition, communication technologies supported by the transceiver may include global system for mobile communication (GSM), code division multi access (CDMA), long term evolution (LTE), wireless-fidelity (Wi-Fi), Bluetooth^{™}, radio frequency identification (RFID), infrared data association (IrDA), Zigbee, near field communication (NFC), and the like. The input unit may be, for example, a conventional keypad or a keyboard, a mouse, a microphone receiving a voice signal, a camera, or various other types of input means that detects or receives various forms of user input. The output unit may be, for example, a display screen that outputs an image, a speaker that outputs sound, a haptic device that generates vibration, or various other output means.

The user device 140 shown in FIG. 2 may provide information. The user device 140 may provide first biometric data of the user of the sensor. Following provision of the first biometric data, the user device 140 may identify a noise segment in the first biometric data. The noise segment may correspond to a segment in which the sensor is determined to be in a compressed state based on the sensor sensitivity. The user device 140 may provide second biometric data based on the identified noise segment. The second biometric data may be generated by modifying at least one value corresponding to the noise segment in the first biometric data.

The processor 141 may be configured to control overall operation of the user device 140. For example, the processor 141 may control the user device 140 by executing programs stored in the memory 142 of the user device 140. The processor 141 may be implemented as a central processing unit (CPU), a graphics processing unit (GPU), an application processor (AP), or the like, provided within the user device 140, but this is merely an example.

The memory 142, as hardware configured to store various types of data processed by the user device 140, may store data that has been processed or to be processed by the user device 140. Also, the memory 142 may store applications and drivers driven by the user device 140. The memory 142 may include a random-access memory (RAM), such as dynamic random-access memory (DRAM) and static random-access memory (SRAM), read -only memory (ROM), electrically erasable programmable read-only memory (EEPROM), CD-ROM, Blu-ray or other optical disc storage, hard disk drive (HDD), solid state drive (SSD), or flash memory.

The method for providing information of the present disclosure performed by the user device 140 illustrated in FIG. 2 may also be implemented by a non-transitory computer-readable storage medium (or a non-transitory recording medium) for the operation thereof. The method for providing information may be implemented as a software module or algorithm, and may be stored on a computer-readable recording medium as computer-readable code or program instructions executable by the processor 141. Here, the computer-readable recording medium may be a magnetic storage (such as a read-only memory (ROM), a random-access memory (RAM), a floppy disk, and a hard disk), an optical storage (such as a CD-ROM, and a digital versatile disc (DVD)), and the like. The computer-readable recording medium may be distributed across computer systems connected by a network, such that computer-readable code may be stored and executed in a distributed manner. The medium may be read by a computer, stored in a memory, and executed by the processor 141.

FIG. 3 is a flowchart illustrating a method for providing information according to an example embodiment.

Specifically, FIG. 3 is a flowchart illustrating a method for providing information performed by an electronic device according to an example embodiment. Here, the electronic device may correspond to the user device 140.

In operation S310, the user device 140 may provide first biometric data of a user of the sensor. The first biometric data may include information related to blood glucose level of the user detected by a sensor corresponding to a user terminal. The user device 140 may display the first biometric data of the user via a display unit of the user terminal. Here, the description of the aforementioned body-attachable unit 110 may apply to the sensor.

In this case, even if the first biometric data is modified under a specific condition such as the sensor being subjected to pressure, the user device 140 may still provide the relevant information without delaying the output of the first biometric data.

In operation S320, subsequent to providing the first biometric data, the user device 140 may identify a noise segment in the first biometric data based on the sensor sensitivity.

The sensor sensitivity may be determined based on a conversion function defining a relationship between an electrical current value measured from blood of the user and a blood glucose level corresponding to the electrical current value. The conversion function may be determined or extracted from the computing component 130 to acquire the blood glucose level. The conversion function may correspond to a parameter indicating the responsiveness of the sensor to changes in glucose concentration and may be expressed as a linear or nonlinear model.

In an example embodiment, the noise segment may correspond to a segment in which the sensor is determined to be in a compressed state. The noise segment determined to be in the compressed state may correspond to a segment in which the sensor sensitivity is maintained at or below a threshold value for a reference duration or longer. The reference duration and the threshold value may correspond to predetermined values. For example, the user device 140 may identify a segment as the noise segment determined to be in the compressed state when the sensor sensitivity remains at or below Y1 nA/mM for X1 minutes or longer.

In an example embodiment, the reference duration and the threshold value may be adaptively adjusted according to a disease severity level determined based on historical biometric data of the user. For example, if the disease severity level determined by analyzing historical biometric data is equal to or greater than the reference level, indicating a high-risk group, the user device 140 may adaptively adjusted to identify a segment where sensor sensitivity maintained at or below Y2 nA/mM for at least X2 minutes as the compressed state.

In an example embodiment, the sensor sensitivity may be determined based on a slope of the conversion function derived from the biometric data. However, using the slope of the conversion function to determine the sensor sensitivity is merely an example, and the scope of the present specification is not limited thereto, and example embodiments in which the sensor sensitivity is determined by various methods may also be included. Based on statistical data including the disease severity level of the user, X2 and Y2 may be adjusted to be greater or smaller than X1 and Y1. For example, when the disease severity level is determined to be equal to or greater than the reference level, indicating a high-risk group, X2 may be adjusted to be greater than X1, and Y2 to be smaller than Y1. Thus, the determination of the noise segment may be adaptively performed based on the disease severity level of the user. Here, the reference level may be predetermined based on various factors associated with diabetes, such as the user's age and family medical history.

In operation S330, the user device 140 may provide second biometric data by modifying at least one value corresponding to the noise segment in the first biometric data. The second biometric data may include information related to the user's blood glucose level detected by the sensor corresponding to the user terminal. The user device 140 may display the second biometric data of the user of the sensor via a display unit of the user terminal.

In the present specification, the second biometric data may include at least one biometric data value modified by interpolating at least one value corresponding to the noise segment in the first biometric data, along with biometric data on which the interpolation is not performed in segments other than the noise segment. Alternatively, the second biometric data may correspond to biometric data generated by modifying at least one value corresponding to the noise segment in the first biometric data via interpolation, while biometric data for which the interpolation is not performed in segments other than the noise segment corresponds to the first biometric data, and the first biometric data and the second biometric data may be provided together.

In an example embodiment, the user device 140 may modify the at least one value corresponding to the noise segment in the first biometric data via interpolation and generate the second biometric data including the at least one value modified via interpolation from the noise segment. For example, the user device 140 may interpolate at least one value corresponding to the noise segment based on the sensor sensitivity measured immediately preceding the noise segment and the sensor sensitivity measured immediately following the noise segment. Additionally, various types of interpolation may be performed to modify at least one value corresponding to the noise segment.

In an example embodiment, the user device 140 may provide the second biometric data in which a representation method for distinguishing the noise segment from a segment outside the noise segment in the first biometric data is applied. For example, the user device 140 may provide the second biometric data such that a value corresponding to the noise segment is presented in a first color and a value corresponding to a segment outside the noise segment is presented in a second color. That is, the noise segment is presented in a color different from the segments outside the noise segment in the second biometric data such that a segment including a modified blood glucose level is visually distinguished. In addition to color-coding, various other representation methods may also be applied to distinguish the noise segment from segments outside the noise segment.

In an example embodiment, the user device 140 may provide a notification to the user when the disease severity level of the user, determined based on historical biometric data of the user, is equal to or greater than the reference level. For example, when the disease severity level is determined to be equal to or greater than the reference level, indicating a high-risk group, the user device 140 may provide a notification upon identifying the noise segment. That is, the notification may be provided exclusively to a high-risk group user whose disease severity level equals to or greater than the reference level, to prevent unnecessary disturbance during sleep by avoiding providing a notification every time a noise segment is identified. Here, the notification may be provided as a visual alert displayed on a display unit of the user device 140 or as an audible alert emitted through a speaker of the user device 140.

More specifically, the user device 140 may provide a notification to the user when the disease severity level of the user, determined based on historical biometric data of the user, is equal to or greater than the reference level, and at least one modified value included in the second biometric data exceeds a threshold value. That is, even when the disease severity level of the user is determined to be equal to or greater than the reference level, the user device 140 may withhold the notification if none of the modified values in the second biometric data satisfies a predetermined threshold value set by the user in advance. Accordingly, by utilizing the threshold value in conjunction with the reference level to determine whether to provide a notification, the possibility of disturbing user's sleep may be reduced. Here, the threshold value may be statistical information set based on various clinical factors such as the user's age, disease severity level, and other health risks to provide a notification regarding the health state of the user while avoiding unnecessary sleep disturbance.

In another example, the user device 140 may provide a notification to the user when the disease severity level of the user, determined based on historical biometric data of the user, is equal to or greater than the reference level, and at least one modified value included in the second biometric data is below a threshold value. Here, the threshold value may be biometric data of a glucose level indicative of a hypoglycemic state.

In an example embodiment, the user device 140 may identify a noise segment in the first biometric data in accordance with a predetermined cycle set based on the disease severity level determined from historical biometric data of the user. For example, if the disease severity level is determined to be equal to or below the reference level, indicating a row-risk group, based on analysis of historical biometric data of the user, the user device 140 may identify a noise segment on an hourly basis and generate the second biometric data by modifying a value corresponding to the noise segment in the first biometric data. Alternatively, if the disease severity level is determined to be equal to or greater than the reference level, indicating a high-risk group, based on analysis of historical biometric data of the user, the user device 140 may identify a noise segment every 20 minutes, and generate the second biometric data by modifying a value corresponding to the noise segment in the first biometric data.

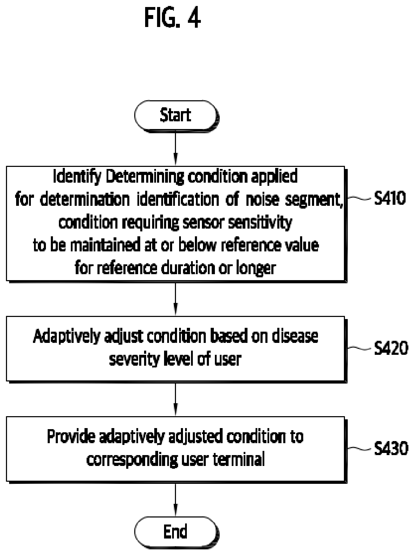

FIG. 4 is a flowchart illustrating a method for providing information performed by a server according to an example embodiment.

In operation S410, a server may identify a condition applied for determination of the noise segment, the condition requiring that the sensor sensitivity is maintained at or below a reference value for a reference duration or longer. Here, the noise segment may correspond to a segment determined as being in a compressed state. In operation S420, the server may adaptively adjust the condition based on the disease severity level of the user. In operation S430, the server may provide the adaptively adjusted condition to a corresponding user terminal. Here, the user terminal may correspond to the user device 140.

That is, the server may maintain a table storing information, including the reference duration and the threshold value related to the condition, based on various factors, retrieve information corresponding to the adaptively adjusted condition based on the disease severity level of the user from the table, and provide the retrieved information to the user terminal.

For example, the server may determine the reference duration and the threshold value associated with the identified condition by referencing the table based on basic information of a user 1, identify information adaptively adjusted in response to changes in the disease severity level of the user, and provide the identified information to a terminal corresponding to the user 1. If there is no change in the disease severity level of the user, the condition remains unchanged. Alternatively, if the disease severity level of the user changes, information associated with a condition that is adaptively adjusted based on the degree of the change may be provided to a user terminal.

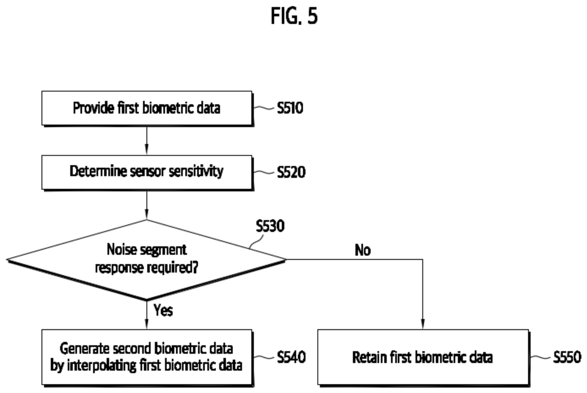

FIG. 5 is a flowchart illustrating a method for providing information performed by a user device according to an example embodiment.

In operation S510, the user device 140 may provide first biometric data. The user device 140 may display the first biometric data of the user detected from the sensor via a display unit. Even if it is confirmed that the first biometric data has changed due to the sensor being subject to pressure, the user device 140 may provide the relevant information without delaying the output of the first biometric data.

In operation S520, the user device 140 may determine the sensor sensitivity. The user device 140 may determine the sensor sensitivity after the first biometric data is provided. The sensor sensitivity may be determined for all segments during which the first biometric data is provided.

In operation S530, the user device 140 may identify a noise segment in the first biometric data determined to be in the compressed state, based on the determined sensor sensitivity.

In operation S540, if it is determined that there is a segment corresponding to the noise segment in the first biometric data based on the sensor sensitivity, the user device 140 may generate second biometric data by interpolating first biometric data included in the identified noise segment. The user device 140 may provide the second biometric data of the user of the sensor via a display unit. The user device 140 may provide the second biometric data of a segment corresponding to the noise segment in a manner different from the method used to provide the first biometric data.

Meanwhile, in operation S550, if there is no noise segment identified in the first biometric data, the user device 140 may retain the first biometric data.

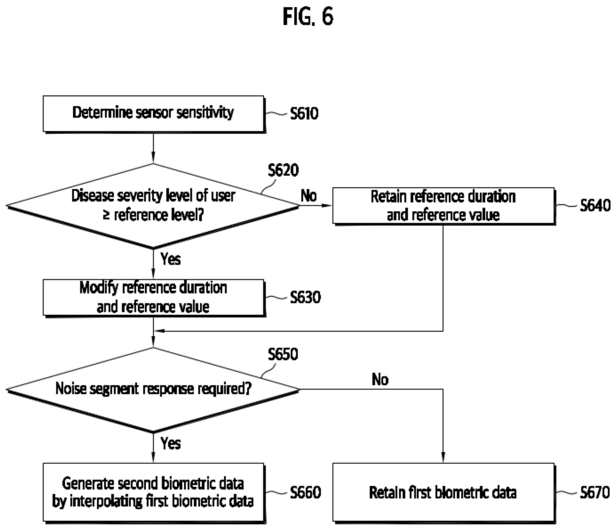

FIG. 6 is a flowchart illustrating a method for providing information performed by a user device according to another example embodiment. The user device 140 may identify the noise segment based on the reference duration and the threshold value set differently according to the disease severity level of the user.

In operation S610, the user device 140 may determine the sensor sensitivity. The user device 140 may determine the sensor sensitivity after the first biometric data is provided.

In operation S620, the user device 140 may analyze historical biometric data of the user to determine whether the disease severity level of the user is equal to or greater than the reference level.

If the disease severity level of the user is equal to or greater than the reference level, the user device 140 may change the reference duration and the threshold value in operation S630. For example, if the disease severity level determined to be equal to or greater than the reference level, indicating a high-risk group, by analysis of historical biometric data of the user, the user device 140 may be adaptively adjusted to identify a segment in which the sensor sensitivity is maintained at a value equal to or below Y2 nA/mM for at least X2 minutes instead of the previous reference time X1 and the reference value Y1 as the noise segment determined to be in the compressed state.

Here, the user device 140 may change the reference duration and the threshold value based on information received from the server or based on other relevant information determined by the user device itself.

Meanwhile, in operation S640, if the disease severity level of the user is below the reference level, the user device 140 may identify a segment as the noise segment determined to be in the compressed state when the sensor sensitivity is maintained at a value equal to or below Y1 nA/mM for at least X1 minutes, consistent with the initial settings.

In operation S650, based on the determined sensor sensitivity, the user device 140 may determine whether to respond to the noise segment in the first biometric data, the noise segment corresponding a state in which the sensor is determined to be in a compressed state.

In operation S660, if a segment corresponding to the noise segment is identified in the first biometric data, the user device 140 may generate the second biometric by modifying first biometric data included in the noise segment via interpolation.

Meanwhile, in operation S670, if there is no noise segment identified in the first biometric data, the user device 140 may retain the first biometric data.

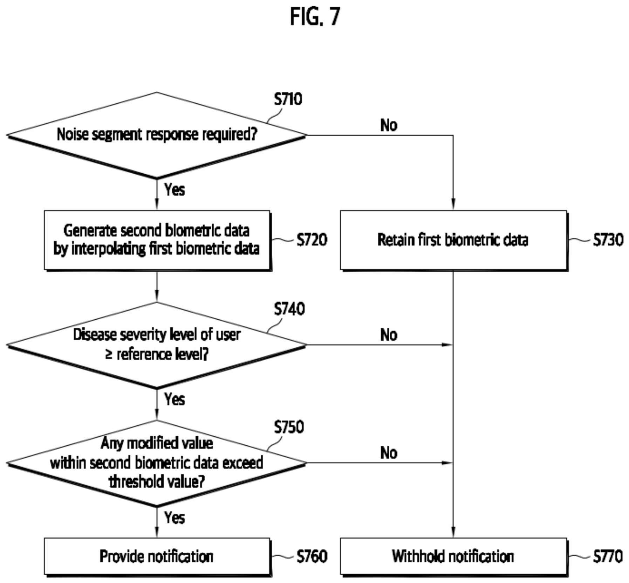

FIG. 7 is a flowchart illustrating a process of providing a notification by a user device according to an example embodiment.

In operation S710, the user device 140 may determine whether there is a segment corresponding to the noise segment in the first biometric data, a period in which the sensor is determined to be in a compressed state, based on a determined sensor sensitivity.

In operation S720, if it is determined that there is a segment corresponding to the noise segment in the first biometric data, the user device 140 may generate second biometric data by modifying first biometric data included in the noise segment via interpolation.

Meanwhile, in operation S730, if there is no noise segment identified in the first biometric data, the user device 140 may retain the first biometric data. If the first biometric data is retained, the user device 140 may not provide a notification to the user in operation S770.

In operation S740, the user device 140 may analyze historical biometric data of the user to determine whether the disease severity level of the user is equal to or greater than the reference level. If the disease severity level of the user is below the reference level, the user device 140 may not provide a notification to the user in operation S770.

In operation S750, if the disease severity level of the user is equal to or greater than the reference level, the user device 140 may determine whether any of the modified values in the second biometric data exceed the threshold value. If no value among the modified values in the second biometric data exceeds the threshold value, the user device 140 may not provide a notification to the user in operation S770.

If at least one value among the modified values in the second biometric data exceeds the threshold value, the user device 140 may provide a notification to the user in operation S760. As such, by determining whether to provide a notification based on both the disease severity level and the threshold value, sleep disturbance caused by notifications may be minimized.

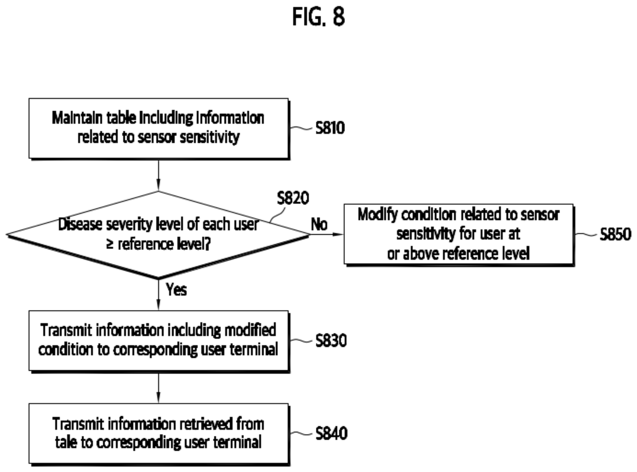

FIG. 8 is a flowchart illustrating a method for providing information performed by a server according to an example embodiment.

In operation S810, the server may maintain a table including information related to the sensor sensitivity. Here, the table may be stored and managed in a memory such as a volatile memory or a non-volatile memory.

In operation S820, the server may analyze historical biometric data of each user to determine whether the disease severity level of the user meets or exceeds the reference level.

In operation S830, if the disease severity level of a user is equal to or greater than the reference level, the server may adjust a condition related to the sensor sensitivity for users with disease severity levels equal to or greater than the reference level by referring to the information maintained in the table. For example, the server may adjust the reference duration and the threshold value used as criteria for determining the noise segment when the disease severity level of a user is equal to or above the reference level.

Then, in operation S840, the server may transmit information including the adjusted condition to a terminal of the user whose disease severity level is equal to or greater than the reference revel.

Meanwhile, in operation S850, if the disease severity level of the user is below the reference level, the server may transmit information retrieved from the table to a terminal corresponding to each user without modification.

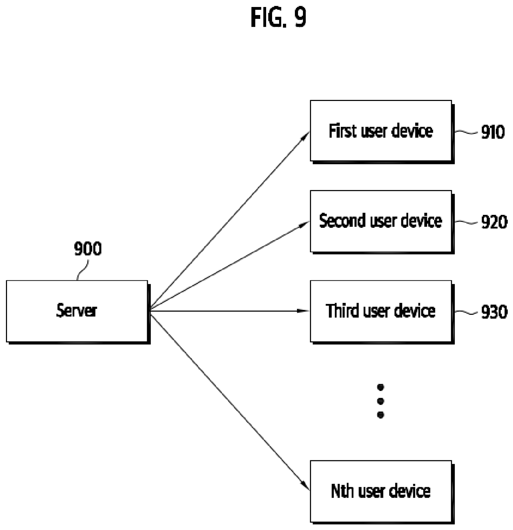

FIG. 9 is a diagram illustrating a relationship between a server and a user device according to an example embodiment.

The server 900 may retrieve information related to the sensor sensitivity from the table based on whether disease severity level of the user is equal to or greater than the reference level and transmit the information to user devices 910, 920, and 930.

For example, when the disease severity level of a first user or a third user is below the reference level, the server may transmit information retrieved from the table based on each user's previous information to a first user device 910 or a third user device 930. As another example, when the disease severity level of the second user is equal to or greater than the reference level, the server may retrieve information related to modified sensor sensitivity from the table based on the disease severity level of a second user and transmit the information to a second user device 920.

However, although example embodiments of the present disclosure have been described with reference to the accompanying drawings and specific terminology, they are provided in an illustrative sense and for descriptive purposes only. The specific embodiments and terms are not intended to limit the scope of the disclosure as defined by the claims. It will be apparent to those skilled in the art that other modifications and variations based on the technical spirit of the present disclosure can be implemented without departing from the scope of the invention.

The server or terminal according to the above-described example embodiments may include a processor, a memory for storing program data and executing the stored program data, a permanent storage such as a disk drive, a communications port for communicating with external devices, and a user interface device such as a touch panel, a key or a button. Methods implemented as software modules or algorithms may be stored on a computer-readable recording medium as computer-readable code or program commands which may be executed by the processor. Here, the computer-readable recording medium may include a magnetic storage (such as a read-only memory (ROM), a random-access memory (RAM), a floppy disk, and a hard disk), an optical storage (such as a CD-ROM, and a digital versatile disc (DVD)), or the like. The computer-readable recording medium may be distributed across computer systems connected by a network, such that computer-readable code may be stored and executed in a distributed manner. The medium may be read by a computer, may be stored in a memory, and may be executed by the processor.

The example embodiments may be represented by functional blocks and various processing steps. Such functional blocks may be realized by any number of hardware and/or software components configured to perform specified functions. For example, the example embodiments may adopt direct circuit configurations such as a memory, a processor, a logic circuit, and a look-up table that may execute various functions by control of one or more microprocessors or other control devices. As with elements executed by software programming or software elements, the example embodiments may be implemented by programming or scripting languages such as C, C++, Java, and assembler including various algorithms implemented by combinations of data structures, processes, routines, or of other programming configurations. Functional aspects may be implemented by algorithms executed by one or more processors. In addition, the example embodiments may adopt the related art for electronic environment setting, signal processing, and/or data processing, for example. The terms "mechanism", "element", "means", and "configuration" may be widely used and are not limited to mechanical and physical components. These terms may include meaning of a series of routines of software in association with a processor, for example.

The example embodiments described above are merely examples and other embodiments may be implemented within the scope of the following claims.

## Claims

1. A method for providing information performed by an electronic apparatus, the method comprising:
providing (S310) first biometric data of a user of a sensor;
determining (S320), subsequent to providing the first biometric data, a noise segment in the first biometric data based on a sensor sensitivity; and
providing (S330), based on the determination of the noise segment, second biometric data in which at least one value corresponding to the noise segment in the first biometric data is modified.

2. The method of claim 1, wherein the noise segment corresponds to a segment in which the sensor is determined to be in a compressed state,
wherein the noise segment determined to be in the compressed state corresponds to a segment in which the sensor sensitivity is maintained at or below a reference value for a reference duration or longer.

3. The method of claim 2, wherein the reference value and the reference duration are adaptively adjusted (S630) in view of a disease severity level determined based on historical biometric data of the user.

4. The method of any one of claims 1 to 3, wherein the providing (S330) of the second biometric data comprises:
modifying the at least one value corresponding to the noise segment in the first biometric data via interpolation; and
providing (S540, S660, S720) second biometric data including the at least one value modified via the interpolation in the noise segment,
wherein the providing (S330) of the second biometric data comprises providing second biometric data in which a representation method for distinguishing the noise segment from a segment outside the noise segment in the first biometric data is applied to the noise segment.

5. The method of any one of claims 1 to 4, wherein the providing (S330) of the second biometric data comprises providing (S760) a notification to the user when a disease severity level determined based on historical biometric data of the user is at or above a reference level.

6. The method of claim 5, wherein the providing (S760) of the notification comprises providing a notification to the user when the disease severity level determined based on the historical biometric data of the user is at or above the reference level and at least one modified value in the second biometric data is at or above a threshold value (S750).

7. The method of any one of claims 1 to 6, wherein the sensor sensitivity is determined (S520, S610) based on a function defining a relationship between an electrical current value measured from blood of the user and a blood glucose level corresponding to the electrical current value.

8. The method of any one of claims 1 to 7, wherein the determining (S320) of the noise segment comprises determining the noise segment in the first biometric data in accordance with a predetermined cycle set in view of a disease severity level determined based on historical biometric data of the user.

9. An electronic apparatus (140) for providing information, the apparatus comprising:
a processor (141); and
at least one memory (142) configured to store at least one instruction,
wherein, when the at least one instruction is executed, the processor (141) is configured to:
provide first biometric data of a user of a sensor;
determine, subsequent to providing the first biometric data, a noise segment in the first biometric data based on a sensor sensitivity; and
provide, based on the determination of the noise segment, second biometric data in which at least one value corresponding to the noise segment in the first biometric data is modified.

10. The apparatus of claim 9, wherein the noise segment corresponds to a segment in which the sensor is determined to be in a compressed state,
wherein the noise segment determined to be in the compressed state corresponds to a segment in which the sensor sensitivity is maintained at or below a reference value for a reference duration or longer,
wherein the reference value and the reference duration are adaptively adjusted in view of a disease severity level determined based on historical biometric data of the user.

11. The apparatus of claim 9 or 10, wherein the processor, when providing the second biometric data, is configured to:
modify the at least one value corresponding to the noise segment in the first biometric data via interpolation; and
provide second biometric data including the at least one value modified via the interpolation in the noise segment.

12. The apparatus of claim 11, wherein the processor, when providing the second biometric data, is configured to provide second biometric data in which a representation method for distinguishing the noise segment from a segment outside the noise segment in the first biometric data is applied to the noise segment.

13. The apparatus of any one of claims 9 to 12, wherein the sensor sensitivity is determined based on a function defining a relationship between an electrical current value measured from blood of the user and a blood glucose level corresponding to the electrical current value.

14. The apparatus of any one of claims 9 to 13, wherein the processor, when determining the noise segment, is configured to determine the noise segment in the first biometric data in accordance with a predetermined cycle set in view of a disease severity level determined based on historical biometric data of the user.

15. A method for providing information performed by a server (900), the method comprising:
identifying (S410, S810) a condition applied for determination of a noise segment, the condition requiring a sensor sensitivity is maintained at or below a reference value for a reference duration or longer;
adaptively adjusting (S420, S830) the condition in view of a disease severity level of a user; and
providing (S430, S840) the adaptively adjusted condition to a corresponding user terminal (910, 920, 930).
